# EUROPEAN PATENT SPECIFICATION

(11) **EP 2 687 521 B1**
(45) Date of publication and mention of the grant of the patent: **07.01.2015**
(21) Application number: 12177209.9
(22) Date of filing: 20.07.2012
(51) Int. Cl.: C07D 317/24

(54) **"Process for the enantioselective synthesis of landiolol"**
Verfahren zur enantioselektiven Synthese von Landiolol
Procédé de synthèse énantiosélective de landiolol

(43) Date of publication of application: 22.01.2014
(73) Proprietor: Procos S.p.A., 28062 Cameri NO (IT)
(72) Inventor: Garavaglia, Fabio, 28065 Cerano (NO) (IT); Roletto, Jacopo, 10135 Torino (IT); Paissoni, Paolo, 10040 Druento (TO) (IT)
(74) Representative: Minoja, Fabrizio

(56) References cited:
- IGUCHI S ET AL: "DEVELOPMENT OF A HIGHLY CARDIOSELECTIVE ULTRASHORT-ACTING BETA-BLOCKER ONO-1101", CHEMICAL AND PHARMACEUTICAL BULLETIN,, vol. 40, no. 6, 1 January 1992 (1992-01-01), pages 1462-1469, XP002546967, ISSN: 0009-2363
- READY J M: "Asymmetric catalytic synthesis of alpha-aryloxy alcohols: kinetic resolution of terminal epoxides via highly enantioselective ring-opening with phenols", JOURNAL OF THE AMERICAN CHEMICAL SOCIETY, vol. 121, no. 25, 30 June 1999 (1999-06-30), XP002122951, AMERICAN CHEMICAL SOCIETY, WASHINGTON, DC; US ISSN: 0002-7863, DOI: 10.1021/JA9910917
- READY JOSEPH M ET AL: "Highly active oligomeric (salen)Co catalysts for asymmetric epoxide ring-opening reactions", JOURNAL OF THE AMERICAN CHEMICAL SOCIETY, vol. 123, no. 11, 1 January 2001 (2001-01-01), pages 2687-2688, XP002581171, AMERICAN CHEMICAL SOCIETY, WASHINGTON, DC; US ISSN: 0002-7863, DOI: 10.1021/JA005867B [retrieved on 2001-02-23]

## Description

### SUMMARY OF THE INVENTION

The present invention relates to a process for the preparation of Landiolol **1,** ((S)-2,2-dimethyl-1,3-dioxolan-4-yl)methyl 3-(4-((S)-2-hydroxy-3-(2-(morpholine-4-carboxamido)ethylamino)propoxy)phenyl)propanoate) or of its hydrochloride salt **2,** starting from (S)-(2,2-dimethyl-1,3-dioxolan-4-yl)methyl 3-(4-hydroxyphenyl)propanoate **3,** an alkylating agent with epoxide structure **4** and 2-(morpholine-4-carboxamido)ethanamine in the form of the free base or of a salt thereof.

According to a particularly preferred embodiment of the invention, Landiolol is prepared from (S)-(2,2-dimethyl-1,3-dioxolan-4-yl)methyl 3-(4-((R)-3-chloro-2-hydroxypropoxy)phenyl)propanoate, which is in turn prepared from (S)-(2,2-dimethyl-1,3-dioxolan-4-yl)methyl 3-(4-hydroxyphenyl)propanoate **3** through kinetic resolution of epichlorohydrin.

### BACKGROUND OF THE INVENTION

Landiolol **1** is a potent cardioselective beta-blocker with ultrarapid action, used as an arrhythmic agent in the form of the hydrochloride salt.

The synthesis of Landiolol **1** is disclosed in US 5013734, JP 3302647, CN 100506814, JP 2539734 and Chemical & Pharmaceutical Bulletin 1992, 40 (6) 1462-1469. The main synthetic route for the preparation of Landiolol is reported in the following scheme:

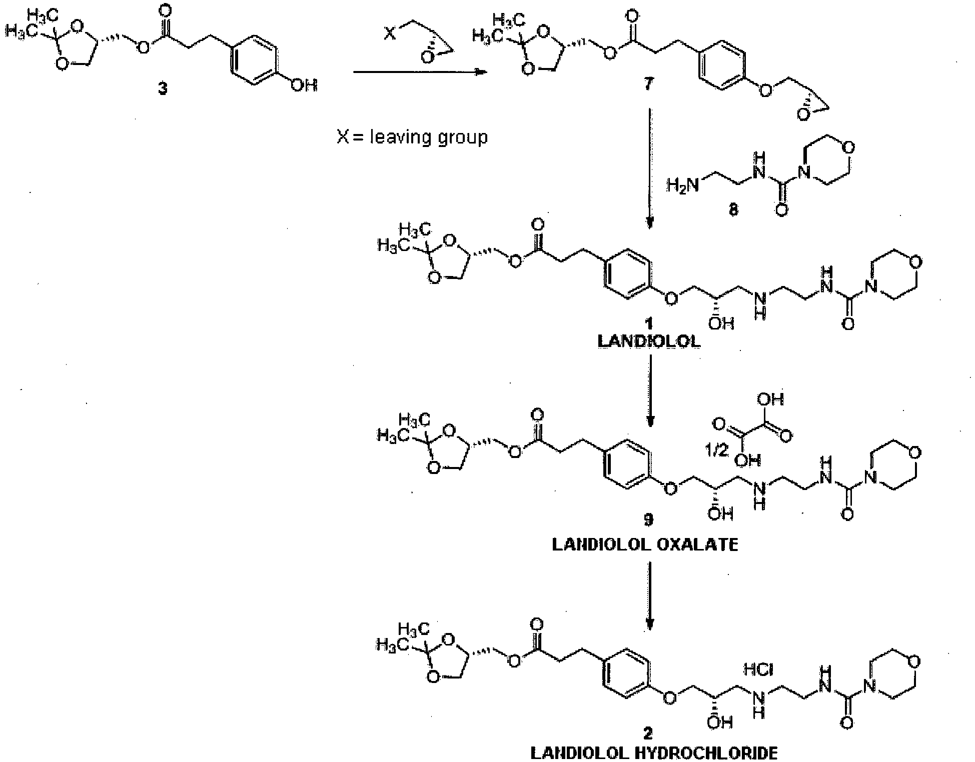

The synthesis of Landiolol **1** is carried out by reacting epoxide **7** with amine **8.** Intermediate **7** is in turn synthesized starting from **3** by reaction with a chiral synthon. US 5013734 discloses the synthesis of **7** from **3** and (S)-epibromohydrin **10.**

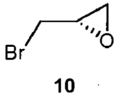

This reagent is very expensive and hardly available on the market. The reaction lasts 16 hrs under refluxing acetone and yields are not very high (76.2%). Furthermore, the reaction between **8** and **7** provides Landiolol **1** in low yields (43.3%), and **1** is then interconverted to intermediate **9** (38.4%, overall yield from **7).** In this final step chloroform is used as the solvent. Moreover, both intermediate **7** and Landiolol **1** require chromatographic purification. JP 3302647 discloses the same synthesis of intermediate **7** but replacing (S)-epibromohydrin **10** with (S)-glycidyl nosylate **11.**

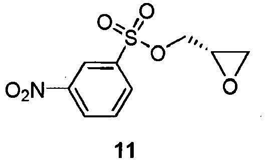

Compound **11** is also very expensive and hardly available on the market, so that no particular advantages are obtained. Moreover, the reaction to give **7** is carried out with sodium hydride in dimethylformamide at room temperature, i.e. not under conditions that are not industrially scalable having caused a number of incidents in the past, due to strong self-heating of the reaction mixture (Bretherick, 7^{th} edition, 2007, 1671-1673). In this case also the yields to obtain the Landiolol **1** are low (42.6% yield from **7** to Landiolol **1).** In this case the amine **8** is replaced with the oxalate salt **6.**

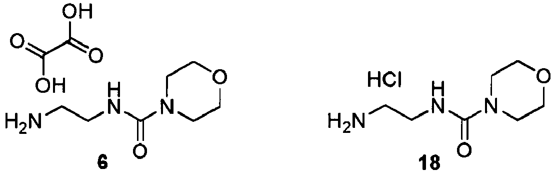

The resulting intermediate **9** (45.8% yield) is then inter-converted to Landiolol **1.** CN 101768148 discloses a substantially identical synthesis to the above one, in which yields from **7** and free base **8** to give Landiolol oxalate **9** are still very low (27.8%). CN 100506814 discloses another synthesis similar to the above described ones, in which (S)-epichlorohydrin (compound of formula **12** in which X is chlorine), which is more easily available on market, is used as the chiral synthon.

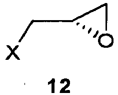

However, this reactive is also very expensive and the reaction is long (16-24 h). Furthermore, the final product Landiolol hydrochloride **2** is obtained by filtration from a water-sodium carbonate-sodium chloride/ether-hexane biphasic mixture, which is not optimal for the industrial production. Chemical & Pharmaceutical Bulletin 1992, 40 (6) 1462-1469 describes the synthesis of 7 starting from 3 and an analogous chiral synthon 13**.**

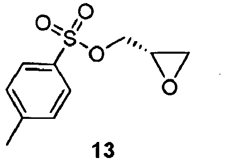

(S)-Glycidyl tosylate **13** is also quite expensive and hardly available on the market and the reaction is time-consuming (15 h). Moreover, in this case also both intermediate **7** and Landiolol **1** require chromatographic purification. In JP 2539734 the compound **7** is synthesized according to the following synthetic scheme:

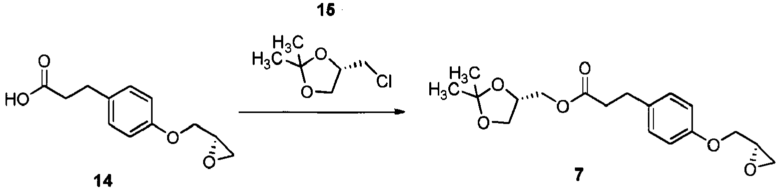

This solution however does not involve any particular advantages. Moreover compound **14** is not commercially available, and one of the expensive chiral synthons described above has to be employed.

The processes described above involve isolation of all of the intermediates and a number of chromatographic purifications, thus negatively affecting yield and productivity, with strong impaction of labor costs on the finished product. In addition, in many cases the reaction of **7** to Landiolol **1** or Landiolol oxalate **9** provides low yields. Furthermore, reaction times are quite long and, most of all, the reagents used are very expensive, hardly available on the market, and cannot be recycled in the process.

### DISCLOSURE OF THE INVENTION

It has now been found that Landiolol **1** and Landiolol hydrochloride **2** can be conveniently prepared according to a novel synthetic procedure as reported hereinbelow:

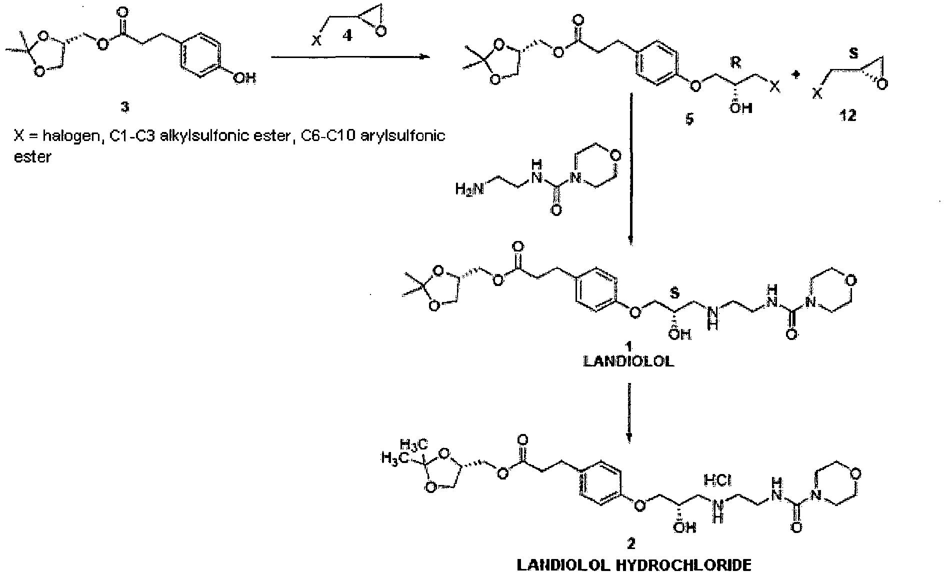

The process of the invention comprises:
a) reaction of (S)-(2,2-dimethyl-1,3-dioxolan-4-yl)methyl 3-(4-hydroxyphenyl)propanoate **3** with a very low cost epoxide alkylating agent of formula **4** in which X is a halogen or a C₁-C₃ alkylsulfonic or C₆-C₁₀ arylsulfonic ester in the presence of an enantioselective catalyst which is easily commercially, quite inexpensive and recyclable, to obtain a compound of formula 5 in which X is as defined above, along with the starting epoxide alkylating agent enriched in the (S) isomer, of formula **12,** with a mild, rapid reaction;
b) reaction of compound of formula **5** obtained in step a) in the presence of a base with 2-(morpholine-4-carboxamido)ethanamine in the form of free base or of a salt thereof to give Landiolol **1** in good yields;
c) salification of Landiolol **1** to give Landiolol hydrochloride **2** with high enantiomeric excess.

In compounds of formula **5** the carbon atom bearing the secondary alcohol group has R stereochemistry.

Preferred examples of compounds of formula **4** are epichlorohydrin, epibromohydrin, glycidyl nosylate, glycidyl tosylate and the like.

A particularly preferred example of compounds of formula **4** is epichlorohydrin.

The enantioselective catalyst I preferably a metal based catalyst, such as a catalyst based on cobalt, manganese, aluminium, copper, samarium, chromium, vanadium and the like, bound to an enantiopure chelating ligand, preferably a salen ligand, such as (R,R)-Bis(3,5-di-*tert*-butylsalicylidene)-1,2-ciclohexanediamine. The enantioselective catalyst is preferably activated with a polar compound, preferably a carboxylic acid, a ionic salt or a multi-fluorinated compound, such as acetic acid, 4-nitrobenzoic acid, cobalt chloride, zinc nitrate, gallium chloride, 3,5-diflurophenol, perfluoro-*tert-*butanol and the like.

In this case, the synthesis of the compounds of formula **5** is carried out in an organic solvent, preferably aprotic polar, such as methyl *tert*-butyl ether (MTBE), dichloromethane, acetonitrile and mixtures thereof, in the presence of an enantioselective catalyst, preferably (R,R)-N,N'-Bis(3,5-di-*tert-*butylsalicylidene)-1,2-ciclohexanediamino cobalt **16,** a polar activator, preferably 4-nitrobenzoic acid **17** and an epoxy alkylating agent, preferably epichlorohydrin.

When epichlorohydrin is used as compound of formula **4,** the compound of formula **5** is obtained in which X is chlorine, (S)-(2,2-dimethyl-1,3-dioxolan-4-yl)methyl 3-(4-((2R)-3-chloro-2-hydroxypropoxy)phenyl)propanoate.

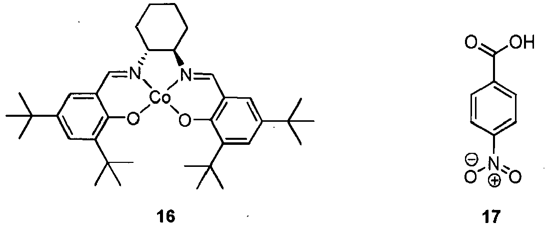

The synthesis of Landiolol **1** is carried out using 2-(morpholine-4-carboxamido)ethanamine as base or a salt thereof, such as the oxalate or the chloride. The reaction is carried out in an organic polar solvent, e.g. an alcoholic solvent, such as isopropanol and isobutanol or preferably acetonitrile, in the presence of a base, preferably an inorganic one, such as potassium carbonate, sodium carbonate, NaOH, KOH and the like and of a ionic, preferably inorganic, catalyst such as KI, NaI, KBr, NaBr and the like.

Salification to Landiolol hydrochloride **2** is carried out using a chlorinated, preferably inorganic, acid such as hydrochloric acid, ammonium chloride and the like. The reaction is carried out in a polar solvent such as isopropanol, acetone, acetonitrile, diisopropyl ether and mixtures thereof.

Intermediate **5** in which X is chlorine is novel and is a further object of the present invention.

According to a preferred embodiment of the invention, the process is carried out as follows:

### Step a

Typically, to activate the salen catalyst, preferably (R,R)-N,N'-bis(3,5-di-*tert*-butylsalicylidene)-1,2-ciclohexanediamino cobalt **16** is reacted with 1.0 ÷ 3.0 equivalents of a carboxylic acid, preferably 4-nitrobenzoic acid **17,** preferably 1.5 ÷ 2.5 equivalents. The reaction is carried out in a polar aprotic solvent, preferably dichloromethane, at a temperature of 10 ÷ 40°C, preferably at a temperature of 20 ÷ 30°C. 4 ÷ 15 Volumes of solvent are used, preferably 7 ÷ 12 volumes with respect to the amount of (R,R)-N,N'-bis(3,5-di-*tert-*butylsalicylidene)-1,2-ciclohexanediamino cobalt **16.** After a dark brown color appears, the solvent is removed thereby obtaining the catalyst the in active form. This is then added with 10 ÷ 100 equivalents of starting product (S)-(2,2-dimethyl-1,3-dioxolan-4-yl)methyl 3-(4-hydroxyphenyl)propanoate **3,** preferably 20 ÷ 50 equivalents, then with a polar aprotic solvent, preferably methyl *tert*-butyl ether (MTBE). 1 ÷ 5 Volumes of solvent are used, preferably 2 ÷ 3 volumes with respect to the amount of (S)-(2,2-dimethyl-1,3-dioxolan-4-yl)methyl 3-(4-hydroxyphenyl)propanoate **3.** Afterwards, 2.0 ÷ 3.0 equivalents of a compound of formula **4,** typically epichlorohydrin, are added, preferably 2.0 ÷ 2,5 equivalents. The reaction is carried out at a temperature of 10 ÷ 40°C, preferably at a temperature of 20 ÷ 30°C. The reaction is monitored by UPLC analysis using a C18 column and water / acetonitrile containing 1% formic acid as the eluent phase. After completion of the reaction, water and toluene are added and phases are separated. The organic phase is then distilled to recover (S)-epichlorohydrin and washed with dilute sodium hydroxide. The organic phase is then concentrated to small volume, added with a polar solvent, acetonitrile or methanol, preferably acetonitrile, concentrated again to small volume to remove toluene and finally added with 5 ÷ 30 volumes of a polar solvent, such as acetonitrile or methanol, preferably acetonitrile. The suspension is filtered thus recovering the catalyst and the resulting solution can be directly used in step **b,** or (S)-(2,2-dimethyl-1,3-dioxolan-4-yl)methyl 3-(4-((2R)-3-chloro-2-hydroxypropoxy)phenyl)-propanoate **5** can be isolated as an oil that can be stored at room temperature for some days. In order to obtain (S)-(2,2-dimethyl-1,3-dioxolan-4-yl)methyl 3-(4-((2R)-3-chloro-2-hydroxypropoxy)phenyl)propanoate **5** as an oil, the solution in the polar solvent is added with decolorizing filter aid, the obtained suspension is filtered and the resulting solution is evaporated to dryness. (S)-(2,2-Dimethyl-1,3-dioxolan-4-yl)methyl 3-(4-((2R)-3-chloro-2-hydroxy-propoxy)phenyl)propanoate **5** is obtained as an oil.

### Step b

Typically, (S)-(2,2-dimethyl-1,3-dioxolan-4-yl)methyl 3-(4-((2R)-3-chloro-2-hydroxypropoxy)phenyl)propanoate **5** obtained in step a, either isolated as an oil or directly from the polar solvent solution, is reacted with an inorganic base, preferably potassium carbonate, in an amount of 1.0 ÷ 6.0 equivalents, 3.0 ÷ 4.0 equivalents, in the presence of a ionic inorganic catalyst, preferably potassium iodide, in catalytic amounts (0.05 ÷ 0.20 eq). Thereafter, 2-(morpholine-4-carboxamido)ethanamine as base or a salt thereof, such as the oxalate or the hydrochloride, preferably the oxalate, is added in an amount of 1.0 ÷ 4.0 equivalents, preferably 2.0 ÷ 3.0 equivalents. The reaction is carried out in a polar solvent, preferably acetonitrile, at a temperature 20 ÷ 85°C, preferably 60 ÷ 85°C. 5 ÷ 30 Volumes of solvent are used, preferably 10 ÷ 20 volumes with respect to the amount of (S)-(2,2-dimethyl-1,3-dioxolan-4-yl)methyl 3-(4-((2R)-3-chloro-2-hydroxypropoxy)-phenyl)propanoate **5.** The reaction is monitored by UPLC analysis using a C 18 column and water / acetonitrile containing 1% formic acid as the eluent. After completion of the reaction, ethyl acetate and water are added and the phases separated. The organic phase is then extracted with water at pH 2 ÷ 5, preferably 3 ÷ 4. The phases are separated and the aqueous phase is extracted again with ethyl acetate at pH 8 ÷ 13, preferably 9 ÷ 12. The solvent of the organic phase is then replaced with 2 ÷ 20 volumes of a polar solvent, such as isopropanol, and the resulting solution can be directly used in step **c,** or Landiolol **1** can be isolated. For this purpose, the solvent is removed or replaced with a polar solvent, for example diisopropyl ether, to promote solidification, then the solvent is stripped from the resulting suspension thereby obtaining Landiolol **1** as an oil which solidifies in time.

### Step c

Typically, Landiolol **1** obtained in step **b** directly from the polar solvent solution or by dissolution of the isolated product is directly salified to give Landiolol hydrochloride **2,** preferably with hydrochloric acid. Salification is carried out in a polar solvent, preferably isopropanol, in amounts of 2 ÷ 20 volumes of solvent, preferably 5 ÷ 10 volumes with respect to the amount of Landiolol **1.** After addition of the acid, the solvent is evaporated off and the product is crystallized by adding 1 ÷ 20 volumes of a polar solvent, preferably acetone. The suspension is filtered and the solid is dried at 25 ÷ 35°C under vacuum for 12 hours to obtain Landiolol hydrochloride **2.** The enantiomeric excess of the final product is analyzed using a Chiralcel OD column and hexane / ethanol as the eluent phase containing diethylamine.

The process of the invention is particularly advantageous in that is effected without isolating any intermediates. Intermediate **5** is obtained with high purity in very high yields under very mild reactions conditions. Furthermore, the starting material **4** in which X is chlorine (epichlorohydrin), is very inexpensive and easily commercially available. The catalysts used are commercially available at low costs and can be easily recovered by simple filtration. Surprisingly, the reaction to give Landiolol **1** starting from the novel intermediate **5** in which X is chlorine provides a markedly higher yield than those obtained with most processes mentioned in the background of the invention, which conversely start from intermediate **7.** The resulting Landiolol **1** can be directly converted to Landiolol hydrochloride **2** in good overall yields, with no further purifications neither intermediate steps. The resulting Landiolol hydrochloride **2** has very high enantiomeric purity.

Furthermore, the process of the invention allows to recover (S)-epichlorohydrin **12,** which is a high added value product that can also be used in the synthesis of Landiolol **1** according to the following scheme, to prepare compound 3:

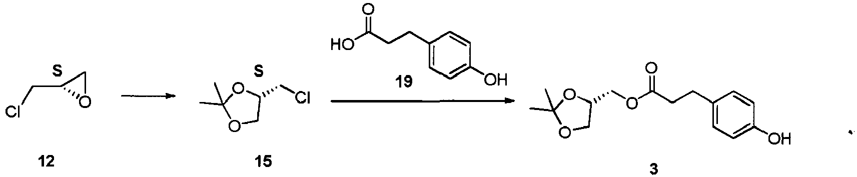

The synthesis of intermediate **15** from **12** in very high yields is described in literature in a number of publications. Some publications which the disclose it are the following: Catalysis Communications, 8(12), 2087-2095; 2007; CN100506814; Journal of Molecular Catalysis A: Chemical, 236(1-2), 72-76; 2005; Chinese Journal of Chemistry, 23(9), 1275-1277; 2005; Synthetic Communications, 35(11), 1441-1445; 2005; Synthetic Communications, 31(22), 3411-3416; 2001; Chemistry Letters, (11), 2019-22; 1990; Khimiya Geterotsiklicheskikh Soedinenii, (1), 33-6; 1991. The synthesis of **3** in high yields starting from **15** and **19** is described in CN100506814. A further publication disclosing it is US5013734. Both publications have already been mentioned in the background of the invention for the synthesis of Landiolol **1.**

The invention is illustrated in detail by the following examples.

### EXPERIMENTAL SECTION

### Example 1: (S)-(2,2-Dimethyl-1,3-dioxolan-4-yl)methyl 3-(4-((2R)-3-chloro-2-hydroxypropoxy)phenyl)propanoate (5)

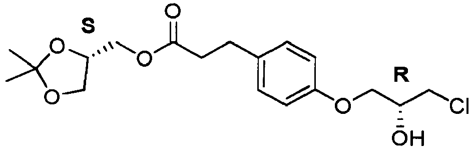

A suspension of (R,R)-N,N'-bis(3,5-di-*tert*-butylsalicylidene)-1,2-ciclohexanediamino cobalt **(16)** (50 mg, 0.0828 mmol) in MTBE (1 ml) is added with acetic acid (10 mg, 0.166 mmol). The mixture is left under stirring for 1 h at 20-25°C until a dark color appears. Afterwards, (S)-(2,2-dimethyl-1,3-dioxolan-4-yl)methyl 3-(4-hydroxyphenyl)propanoate **(3),** (500 mg, 1.78 mmol), then epichlorohydrin (compound of formula **4** in which X is chlorine) (340 mg, 3.56 mmol) are added thereto. The mixture is left under stirring at 20-25°C, monitoring by UPLC. After completion of the reaction, water (5 ml) and toluene (5 ml) are added, the phases are separated and the solvent and (S)-epichlorohydrin are removed from the organic phase under reduced pressure to obtain 600 mg (90.4%) of a dark oil.

### Example 2: (S)-(2,2-Dimethyl-1,3-dioxolan-4-yl)methyl 3-(4-((2R)-3-chloro-2-hydroxypropoxy)phenyl)propanoate (5)

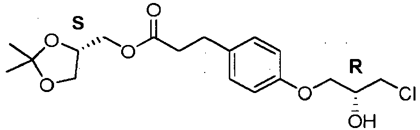

A suspension of (R,R)-N,N'-bis(3,5-di-*tert*-butylsalicylidene)-1,2-ciclohexanediamino cobalt **(16)** (1,9 g, 3.19 mmol) in dichloromethane (20 ml) is added with 4-nitrobenzoic acid **17** (1.1 g, 6.38 mmol). The mixture is left under stirring for 1 h at 20-25°C until a dark color appears. The solvent is replaced with MTBE (30 ml), subsequently (S)-(2,2-dimethyl-1,3-dioxolan-4-yl)methyl 3-(4-hydroxyphenyl)propanoate **(3),** (18 g, 63,8 mmol) and then epichlorohydrin (compound of formula **4** in which X is chlorine) (13,4 g, 140 mmol) are added. The mixture is left under stirring at 20-25°C, monitoring by UPLC. After completion of the reaction, toluene (300 ml) and water (150 ml) are added and the phases are separated. The organic phase is evaporated to dryness thereby recovering the enriched (S)-epichlorohydrin. Toluene (300 ml) and 10% NaOH (100 ml) are added. The phases are separated, the resulting solution is concentrated to a volume of about 50 ml, added with 100 ml of acetonitrile, concentrated to a volume of 50 ml and finally added with 250 ml of acetonitrile. Decolorizing filter aid (2.5 g) is added, the mixture is left under stirring for 15' and the suspension is filtered. The filtrate is evaporated to dryness to obtain 23.7 g (99,6%) of a red-brownish oil.

### Example 3: (S)-(2,2-Dimethyl-1,3-dioxolan-4-yl)methyl 3-(4-((2R)-3-chloro-2-hydroxypropoxy)phenyl)propanoate (5)

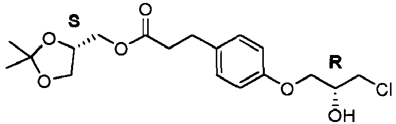

A suspension of (R,R)-N,N'-bis(3,5-di-*tert*-butylsalicylidene)-1,2-ciclohexanediamino cobalt **(16)** (470 mg, 0.780 mmol) in dichloromethane (5 ml) is added with 4-nitrobenzoic acid **17** (270 mg, 1.56 mmol). The mixture is left under stirring for 45' at 20-25°C until a dark color appears. The resulting solution is concentrated to a volume of about 2 ml, added with 5 ml of MTBE, concentrated to a volume of 2 ml and finally added with 6 ml of MTBE, subsequently with (S)-(2,2-dimethyl-1,3-dioxolan-4-yl)methyl 3-(4-hydroxyphenyl)propanoate **(3),** (5 g, 17.7 mmol) and then with epichlorohydrin (compound of formula **4** in which X is chlorine) (3.7 g, 38.9 mmol). The mixture is left under stirring at 20-25°C, monitoring by UPLC. After completion of the reaction, toluene (25 ml) and water (25 ml) are added and the phases are separated. The organic phase is evaporated to dryness thereby recovering the enriched (S)-epichlorohydrin. Acetonitrile (25 ml) is added and the suspension is filtered thereby recovering the catalyst. The resulting solution is concentrated to a volume of about 5 ml, added with 15 ml of toluene, then concentrated to a volume of 5 ml and finally added with 20 ml of toluene and decolorizing filter aid (20.0 g). The mixture is left under stirring for 15' and the suspension is filtered. The filtrate is evaporated to dryness to obtain 6.1 g (92.4%) of a yellow oil.
LC-MS (ESI+) [M+H]⁺ = 373
¹H-NMR (CDCl₃) (chemical shifts expressed in ppm with respect to TMS): 1,37 (3H, s, CH₃); 1,43 (3H, s, CH₃); 2,65 (2H, t, J = 7 Hz, CH₂-Ar); 2,83 (1H, bs, OH); 2,91 (2H, t, J = 7 Hz, CH₂-CO); 3,66 - 3,81 (3H, m, CH in 4 oxolane and CH₂-Cl); 4.00 - 4,25 (6H, m, CH in 4 oxolane, CH₂-OCO, CH₂-OAr and CH in 5 oxolane); 4,25 (1H, m, CH-OH); 6,84 and 7,13 (4H, system AA'XX', aromatics).
¹³C-NMR (CDCl₃) (ppm): 25,3 (CH₃); 26,6 (CH₃); 29,9 (CH₂); 35,8 (CH₂); 45,9 (CH₂-Cl); 64,6 (CH₂); 66,2 (CH₂); 68,5 (CH₂); 69,7 (CH); 73,4 (CH); 109,7; 114,5 (CH); 129,3 (CH); 133,1; 156,7; 172,6 (COOR).
Elemental analysis: C, 58.3%; H, 6.9%; Cl, 9.3%; O, 25.5%. (% calculated: C, 58.0; H, 6.8; Cl, 9.5; O, 25.7).
FT-IR (UATR, cm⁻¹): 3456, 2987, 2936, 1733, 1612, 1512, 1372, 1241, 1154, 1041,828,741,720.

### Example 4: Landiolol (1)

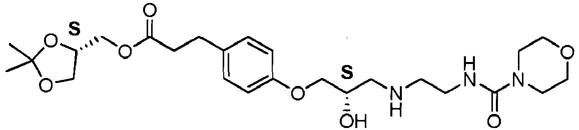

A suspension of (S)-(2,2-dimethyl-1,3-dioxolan-4-yl)methyl 3-(4-((2R)-3-chloro-2-hydroxypropoxy)phenyl)propanoate **(5)** prepared according Example 3 (0.50 g, 0.00134 mol) in isopropanol (10 ml) is added with 2-(morpholine-4-carboxamido)ethanamino hydrochloride **(18)** (1.4 g, 0.00670 mol), heated to 30-35°C and dropwise added with 30% NaOH, keeping pH at 10-11. The mixture is left under stirring at 35-40°C, monitoring by UPLC. After completion of the reaction, ethyl acetate (20 ml) and water (20 ml) are added and the phases are separated. The organic phase is added with water (20 ml) and adjusted to pH 3-4 with hydrochloric acid. The phases are separated and the resulting aqueous phase is then adjusted to pH 10-11 with sodium hydroxide and re-extracted with ethyl acetate (20 ml). The solvent is then evaporated off under reduced pressure to obtain 0.38 g (55.6%) of a pale yellow oil which solidifies in time to a pale yellow solid.

### Example 5: Landiolol (1)

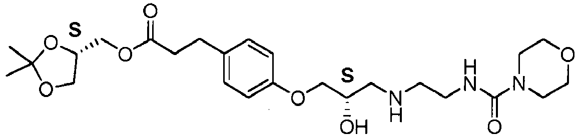

A solution of (S)-(2,2-dimethyl-1,3-dioxolan-4-yl)methyl 3-(4-((2R)-3-chloro-2-hydroxypropoxy)phenyl)propanoate **(5)** prepared according to Example 3 (0.30 g, 0.805 mmol) in acetonitrile (6.0 ml) is added with potassium carbonate 0.45 g (3.22 mmol), and KI 0.013 g (0.0805 mmol), then refluxed for 2 h and added with 2-(morpholine-4-carboxamido)ethanamino oxalate **(6)** (0.64 g, 2.42 mmol). The mixture is refluxed under stirring, monitoring by UPLC. After completion of the reaction, ethyl acetate (10 ml) and water (10 ml) are added and the phases are separated. The organic phase is added with water (10 ml) and adjusted to pH 4-5 with hydrochloric acid, the phases are separated and the resulting aqueous phase is then adjusted to pH 11-12 with sodium hydroxide and re-extracted with ethyl acetate (10 ml). Then the solvent is evaporated off under reduced pressure to obtain 0.29 g (70.7%) of a pale yellow oil which solidifies in time to a pale yellow solid.
LC-MS (ESI+) [M+H]⁺ = 510
¹H-NMR (CDCl₃) (*chemical shifts* expressed in ppm with respect to TMS) (assigned based on the hetero correlation HSQC spectrum): 1.36 (3H, s, CH₃); 1.42 (3H, s, CH₃); 2.63 (2H, t, J = 7 Hz, CH₂-Ar); 2.75 - 2.93 (8H, m, CH₂-CO, CH-CH₂-NH, CH₂-CH₂-NH, NH and OH); 3.35 (6H, m, 2CH₂-N morpholine and CH₂-NH); 3.65 (4H, m, 2CH₂-O morpholine), 3.68 (1H, m, CH in 4 oxolane); 3.94 (2H, bd, CH₂-OAr); 4.00 - 4.20 (4H, m, CH in 4 oxolane, CH₂-OCO and CH in 5 oxolane); 4.25 (1H, m, CH-OH); 5.21 (1H, bt, NH carbamate); 6.83 and 7.11 (4H, system AA'XX', aromatics).
¹³C-NMR (CDCl₃) (ppm) (multiplicity was assigned by DEPT-135): 25.3 (CH₃); 26.6 (CH₃); 29.9 (CH₂); 35.8 (CH₂); 40.2 (CH₂); 43.8 (CH₂-N morpholine); 49.2 (CH₂); 51.5 (CH₂); 64.6 (CH₂); 66.2 (CH₂); 66.4 (CH₂-O morpholine); 68.3 (CH); 70.3 (CH₂); 73.4 (CH); 109.7; 114.4 (CH); 129.2 (CH); 132.8; 157.0; 158.0; 172.5 (COOR).
FT-IR (UATR, cm⁻¹): 3350. 2858, 1735, 1626, 1512, 1454, 1371, 1244, 1153, 1115, 1040. 829, 733.

### Example 6: Landiolol hydrochloride (2)

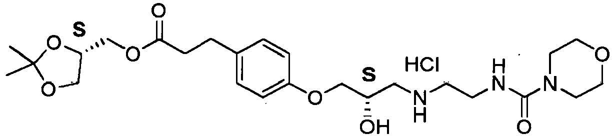

A solution of Landiolol **(1)** prepared according to Example 5 (100 mg, 0.196 mmol) in isopropanol (6.0 ml) is added with 18% isopropanol hydrochloric acid (40 mg, 0.197 mmol). The solvent is then evaporated off under reduced pressure and the residue is crystallized from acetone (2 ml). The suspension is filtered and the crystal is dried at 25°C for 12 h to obtain 80 mg (74.7%) of a white solid.

### Example 7: Landiolol hydrochloride (2) from (S)-(2,2-dimethyl-1,3-dioxolan-4-yl)methyl 3-(4-hydroxyphenyl)propanoate (3)

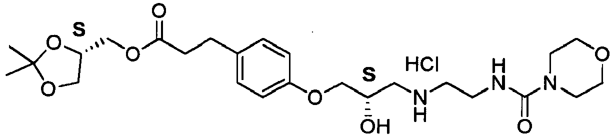

A suspension of (R,R)-N,N'-bis(3,5-di-*tert*-butylsalicylidene)-1,2-ciclohexanediamino cobalt **(16)** (47 mg, 0.0780 mmol) in dichloromethane (1 ml) is added with 4-nitrobenzoic acid 17 (27 mg, 0.156 mmol). The mixture is left under stirring for 45' at 20-25°C until a dark color appears. The resulting solution is concentrated to a volume of about 0.5 ml, added with 0.5 ml of MTBE, concentrated to a volume of 0.5 ml and finally added with 0.5 ml of MTBE, then with (S)-(2,2-dimethyl-1,3-dioxolan-4-yl)methyl 3-(4-hydroxyphenyl)propanoate **(3),** (0.5 g, 1,77 mmol) and then with epichlorohydrin (compound of formula **4** in which X is chlorine) (0.37 g, 3,89 mmol). The mixture is left under stirring at 20-25°C, monitoring by UPLC. After completion of the reaction, toluene (10 ml) and water (10 ml) are added and the phases are separated. The organic phase is evaporated recovering the enriched (S)-epichlorohydrin, then added again with toluene (10 ml) and washed with 10% NaOH (10 ml). The resulting solution is concentrated to a volume of about 2 ml, added with 5 ml of acetonitrile, concentrated to a volume of 2 ml and finally added with 10 ml of acetonitrile). The suspension is filtered thus recovering the catalyst and the solution is added with potassium carbonate 0.79 g (5,64 mmol), and KI 0.026 g (0.161 mmol), refluxed for 2 h, then added with 2-(morpholine-4-carboxamido)ethanamino oxalate **(6)** (1.07 g, 4.03 mmol). The mixture is refluxed under stirring, monitoring by UPLC. After completion of the reaction, ethyl acetate (20.0 ml) and water (20 ml) are added and the phases are separated. The organic phase is then adjusted to pH 4-5 with hydrochloric acid and extracted with water (20 ml). The phases are separated and the resulting aqueous phase is then adjusted to pH 11-12 with sodium hydroxide and re-extracted with ethyl acetate (20 ml). The resulting solution is concentrated to a volume of about 5 ml, added with 20 ml of isopropanol, concentrated to a volume of 5 ml and finally added with 30 ml of isopropanol, then 18% isopropanol hydrochloric acid (0.24 g, 1.18 mmol). The solvent is then evaporated off under reduced pressure and the residue is crystallized from acetone (10 ml). The suspension is filtered and the crystal is dried at 25°C for 12 h to obtain 0.48 g (49.7% total, enantiomeric purity: 99.8%) of a white solid.
m.p.: 126°C (from literature 123-127°C)
LC-MS (ESI+) [M+H]⁺ = 510
FT-IR (UATR, cm⁻¹): 3265, 2941, 2789, 2419, 1723, 1615, 1538, 1515, 1435, 1371, 1260. 1242, 1196, 1118, 1047, 887, 838, 821, 771.

## Claims

1. Process for the preparation of Landiolol hydrochloride with high enantiomeric excess, comprising the following steps:
a) reaction of (S)-(2,2-dimethyl-1,3-dioxolan-4-yl)methyl 3-(4-hydroxyphenyl)propanoate with a compound of formula **4**: 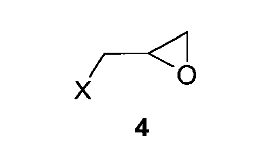 in which X is a halogen or a C₁-C₃ alkylsulfonic or C₆-C₁₀ arylsulfonic ester, in the presence of an enantioselective catalyst containing a metal bound to an enantiopure chelating ligand, to give a compound of formula **5**: 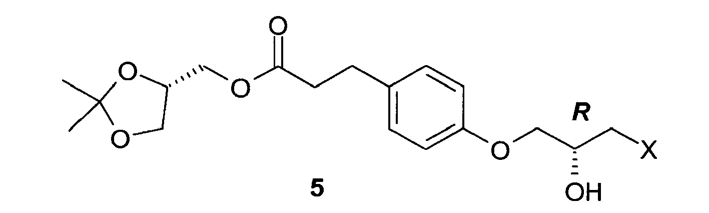 in which X is as defined above, along with the starting compound **4** enriched in the (S) isomer, of formula **12**: 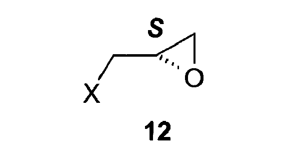 in which X is as defined above;
b) reaction of the compound of formula **5** obtained in step a) in the presence of a base with N-(2-aminoethyl)morpholine-4-carboxamido)ethanamine as the free base or a salt thereof to give Landiolol **1;**
c) salification of Landiolol **1** obtained in step b) to give Landiolol hydrochloride **2.**

2. The process of claim 1 wherein the compound of formula **4** is selected from the group consisting of epichlorohydrin, epibromohydrin, glycidyl nosylate, or glycidyl tosylate.

3. The process as claimed in claim 1 and 2 wherein the compound of formula **4** is epichlorohydrin.

4. The process of claim 1 wherein the enantiopure chelating ligand of the enantioselective catalyst is a salen ligand.

5. The process of claim 1 and 4 wherein the enantioselective catalyst is (R,R)-N,N'-bis(3,5-di-*tert*-butylsalicylidene)-1,2-cyclohexanediamino cobalt.

6. The process of claim 1 and 5 wherein the enantioselective catalyst is activated with a polar compound selected from a carboxylic acid, a ionic salt or a multi-fluorinated compound.

7. The process as claimed in claim 6 wherein the polar compound is selected from acetic acid, 4-nitrobenzoic acid, cobalt chloride, zinc nitrate, gallium chloride, 3,5-diflurophenol, or perfluoro-*tert*-butanol.

8. The process as claimed in any one of the above claims wherein the compound of formula **4** is epichlorohydrin, the enantioselective catalyst is (R,R)-N,N'-bis(3,5-di-*tert*-butylsalicylidene)-1,2-cyclohexanediamino cobalt and the activating polar compound is 4-nitrobenzoic acid.

9. The process as claimed in any one of the above claims wherein the compound of formula **5** and Landiolol are not isolated.

10. The compound of formula **5** as defined in claim 1 wherein X is chlorine, (S)-(2,2-dimethyl-1,3-dioxolan-4-yl)methyl 3-(4-((2R)-3-chloro-2-hydroxy-propoxy)phenyl)propanoate.

## Patentansprüche

1. Verfahren zur Herstellung von Landiolol-Hydrochlorid mit hohem enantiomerem Überschuss, umfassend die folgenden Schritte:
a) Umsetzen von (S)-(2,2-Dimethyl-1,3-dioxolan-4-yl)methyl-3-(4-hydroxyphenyl)propanoat mit einer Verbindung der Formel **4**: 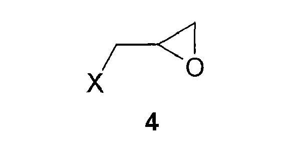 in der X ein Halogen oder ein C₁-C₃-Alkylsulfon- oder C₆-C₁₀-Arylsulfonester ist, in Gegenwart eines enantioselektiven Katalysators, der eine Metallbindung zu einem enantiomerenreinen chelatbildenden Liganden enthält, um eine Verbindung der Formel **5** zu erhalten: 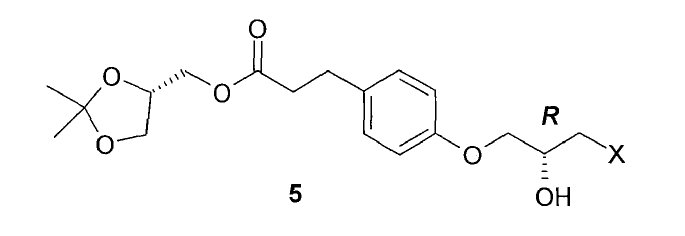 in der X die oben angegebene Definition aufweist, zusammen mit der in dem (S)-Isomer der Formel **12** angereicherten Ausgangsverbindung **4**: 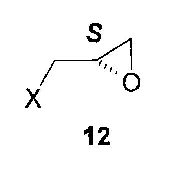 in der X die oben angegebene Bedeutung aufweist;
b) Umsetzen der in Schritt a) erhaltenen Verbindung der Formel **5** in Gegenwart einer Base mit N-(2-Aminoethyl)morpholin-4-carboxamido)ethanamin als die freie Base oder ein Salz davon, wobei Landiolol 1 erhalten wird;
c) Salzbildung des in Schritt b) erhaltenen Landiolols 1, wobei Landiolol-Hydrochlorid **2** erhalten wird.

2. Verfahren nach Anspruch 1, wobei die Verbindung der Formel **4** ausgewählt ist aus der Gruppe bestehend aus Epichlorhydrin, Epibromhydrin, Glycidylnosylat oder Glycidyltosylat.

3. Verfahren nach Anspruch 1 und 2, wobei die Verbindung der Formel **4** Epichlorhydrin ist.

4. Verfahren nach Anspruch 1, wobei der enantiomerenreine chelatbildende Ligand des enantioselektiven Katalysators ein Salen-Ligand ist.

5. Verfahren nach Anspruch 1 und 4, wobei der enantioselektive Katalysator-(R,R)-N,N`-bis(3,5-di-*tert*-butylsalicyliden)-1,2-cyclohexandiamino-cobalt ist.

6. Verfahren nach Anspruch 1 und 5, wobei der enantioselektive Katalysator mit einer polaren Verbindung aktiviert wird, die ausgewählt ist aus einer Carbonsäure, einem ionischen Salz oder einer mehrfach fluorierten Verbindung.

7. Verfahren nach Anspruch 6, wobei die polare Verbindung ausgewählt ist aus Essigsäure, 4-Nitrobenzoesäure, Cobaltchlorid, Zinknitrat, Galliumchlorid, 3,5-Difluorphenol oder Perfluor-*tert*-butanol.

8. Verfahren nach einem der vorhergehenden Ansprüche, wobei die Verbindung der Formel 4 Epichlorhydrin, der enantiosektive Katalysator (R,R)-N,N'-bis(3,5-di-*tert-*butylsalicyliden)-1,2-cyclohexandiamino-cobalt und die aktivierende polare Verbindung 4-Nitrobenzoesäure ist.

9. Verfahren nach einem der vorhergehenden Ansprüche, wobei die Verbindung der Formel **5** und Landiolol nicht isoliert werden.

10. Verbindung der Formel 5 wie in Anspruch 1 definiert, wobei X Chlor ist, (S)-(2,2-dimethyl-1,3-dioxolan-4-yl)methyl 3-(4-((2R)-3-chloro-2-hydroxy-propoxy)phenyl)propanoat.

## Revendications

1. Procédé pour la préparation du chlorhydrate de landiolol avec un excès énantiomère élevé, comprenant les étapes suivantes :
a) réaction du (S)-3-(4-hydroxyphényl)propanoate de (2,2-diméthyl-1,3-dioxolan-4-yl)méthyle avec un composé répondant à la formule **4** : 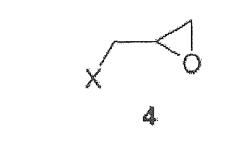 dans laquelle X représente un atome d'halogène ou un ester alkyl(en C₁-C₃)sulfonique ou aryl(en C₆-C₁₀)sulfonique en présence d'un catalyseur énantiosélectif contenant un métal lié à un ligand de chélation énantiopur, pour obtenir un composé répondant à la formule **5** : 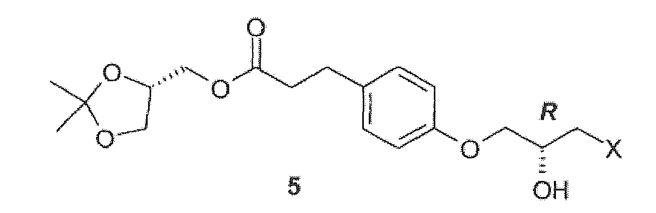 dans laquelle X est tel que défini ci-dessus, conjointement avec le composé de départ **4** enrichi en ce qui concerne l'isomère (S), répondant à la formule **12** : 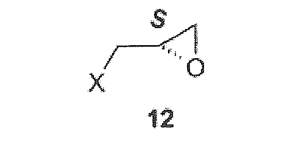 dans laquelle X est tel que défini ci-dessus ;
b) réaction du composé répondant à la formule **5** que l'on obtient à l'étape a) en présence d'une base avec la N-(2-aminoéthyl)morpholine-4-carboxamido)éthanamine sous la forme de la base libre ou d'un de ses sels pour obtenir le landiolol **1** ;
c) salification du landiolol **1** que l'on obtient à l'étape b) pour obtenir le chlorhydrate de landiolol **2**.

2. Procédé selon la revendication 1, dans lequel le composé répondant à la formule **4** est choisi parmi le groupe constitué par l'épichlorhydrine, l'épibromhydrine, le nosylate de glycidyle ou le tosylate de glycidyle.

3. Procédé selon les revendications 1 et 2, dans lequel le composé répondant à la formule **4** est l'épichlorhydrine.

4. Procédé selon la revendication 1, dans lequel le ligand de chélation énantiopur du catalyseur énantiosélectif est un ligand de type salen.

5. Procédé selon les revendications 1 et 4, dans lequel le catalyseur énantiosélectif est le (R,R)-N,N'-bis(3,5-di-tert-butylsalicylidène)-1,2-cyclohexanediamino cobalt.

6. Procédé selon les revendications 1 et 5, dans lequel le catalyseur énantiosélectif est activé avec un composé polaire choisi parmi un acide carboxylique, un sel ionique ou un composé polyfluoré.

7. Procédé selon la revendication 6, dans lequel le composé polaire est choisi parmi l'acide acétique, l'acide 4-nitrobenzoïque, le chlorure de cobalt, le nitrate de zinc, le chlorure de gallium, le 3,5-difluorophénol ou le perfluoro-tert-butanol.

8. Procédé selon l'une quelconque des revendications ci-dessus, dans lequel le composé répondant à la formule **4** est l'épichlorhydrine, le catalyseur énantiosélectif est le (R,R)-N,N'-bis(3,5-di-tert-butylsalicylidène)-1,2-cyclohexanediamino cobalt et le composé polaire d'activation est l'acide 4-nitrobenzoïque.

9. Procédé selon l'une quelconque des revendications ci-dessus, dans lequel le composé répondant à la formule **5** et le landiolol ne sont pas isolés.

10. Composé répondant à la formule **5** tel que défini à la revendication 1, dans lequel X représente un atome de chlore, le (S)-3-(4-((2R)-3-chloro-2-hydroxypropoxy)phényl)propanoate de (2,2-diméthyl-1,3-dioxolan-4-yl)méthyle.
